(19) Europäisches Patentamt
European Patent Office
Office européen des brevets

(11) EP 3 006 030 A1

(12) EUROPEAN PATENT APPLICATION

(43) Date of publication:
**13.04.2016 Bulletin 2016/15**

(51) Int Cl.:
*A61K 31/4453* (2006.01) *A61P 35/00* (2006.01)

(21) Application number: **15188301.4**

(22) Date of filing: **05.10.2015**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA**

(30) Priority: **07.10.2014 KR 20140134964**

(71) Applicant: **Korea Research Institute of Bioscience and Biotechnology**
**Daejeon 34141 (KR)**

(72) Inventors:
- **KWON, Byoung Mog**
  **34141 Daejeon (KR)**
- **HAN, Dong Cho**
  **34141 Daejeon (KR)**
- **LEE, Sangku**
  **34141 Daejeon (KR)**
- **LEE, Yu-Jin**
  **34141 Daejeon (KR)**
- **JEON, Yoon Jung**
  **34141 Daejeon (KR)**
- **CHOI, Jiyeon**
  **34141 Daejeon (KR)**
- **HA, Narum**
  **34141 Daejeon (KR)**
- **HAN, Young Min**
  **34141 Daejeon (KR)**

(74) Representative: **Sonn & Partner Patentanwälte**
**Riemergasse 14**
**1010 Wien (AT)**

(54) **COMPOSITION COMPRISING (S)-(-)-BENPROPERINE FOR PREVENTING OR TREATING CANCER**

(57) The present invention relates to a pharmaceutical composition for preventing or treating cancer, comprising (S)-(-)-benproperine or a pharmaceutically acceptable salt thereof as an active ingredient, and a method for preventing or treating cancer using the same.

【FIG. 3b】

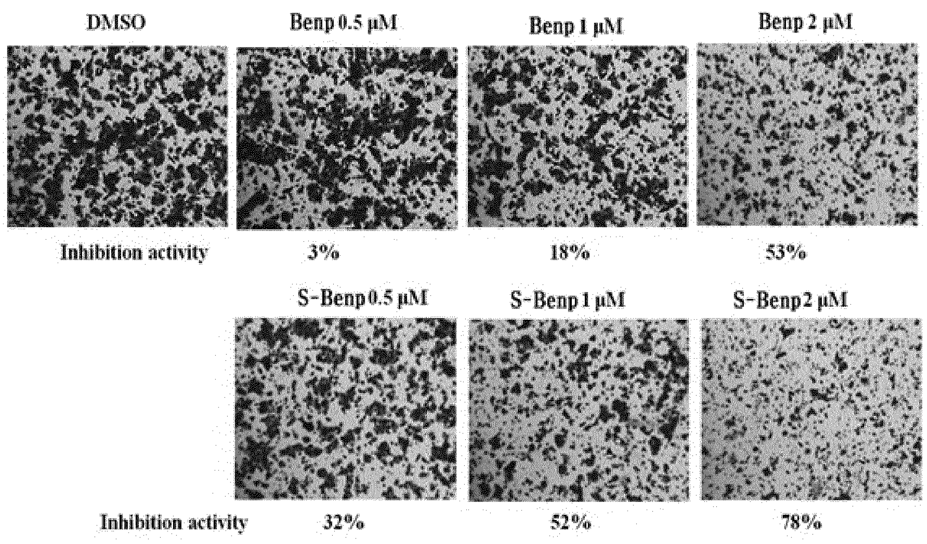




Printed by Jouve, 75001 PARIS (FR)

## Description

### [Technical Field]

The present invention relates to a use of (S)-(-)-benproperine for preventing or treating cancer.

### [Background Art]

The number of cancer deaths has reached 7.6 million, which corresponds to 17% of deaths worldwide, and it is expected that the number of worldwide cancer deaths will rise to 9 million by 2015, and to 11.4 million by 2030. Cancer has been the leading cause of death in Korea, and the societal cost thereof is huge, thus, the development of effective anticancer agents has become more and more necessary.

The main cause of life-threatening cancer lies in the metastasis of cancer cells. Currently, the universal method for treating cancer includes surgery, however cancer cells tend to migrate to other parts of the body from the primary cancer site, and thus the surgery may only be effective in earlier stages of cancer.

Meanwhile, the metastasis of cancer, just like cancer occurrences, occurs due to a combination of various genes and factors that are involved in migration and invasion of cancer cells (Marina Bacac and Ivan Stamenkovic. Annual Review of Pathology: Mechanism of Disease, 3, 221-247, 2008).

Cell migration plays an important role in cancer metastasis, for example, during the migration of cancer cells from the early primary cancer site to the blood via the extracellular matrix (ECM), from a secondary metastatic tissue to outside the blood, or the migration of vascular endothelial cells within new blood vessels. A polarity of migrating cells is induced by an activated signal-receptor, which is induced by a cell migration inducing material. Also, the cell membrane in front of cells is expanded forward by actin polymerization, and the cells adhere to the extracellular matrix via an integrin. Herein, a strong contractile force is generated between actin polymers by myosin linked to the actin polymers, providing the strong contractile force throughout the cell. Accordingly, the direction of cell movement is determined by the difference in adhesive force between the front and back of the cells, pushing the cells forward (Peter Friedl, et al., Nature Cancer Review, 2003, 3: 362). Accordingly, a cell migration inhibitor inhibits the migration of cancer cells to prevent further spread of cancer, and allows for the administration of an anticancer agent, which induces apoptosis of cancer cells in a state of inhibited movement, and thus is recognized as a realistic approach to prolonging the lives of cancer patients.

Meanwhile, benproperine having a chemical name of 1-[1-(2-benzyl phenoxy)propan-2-yl]piperidine, is being used as an anti-tussive agent for cough and nonproductive cough, and is widely known as a less toxic drug which can be administered orally (Siwei Chen, et al., Journal of Pharmacy and Pharmacology, 56,277-280,2004).

Also, the present inventors have discovered that the benproperine is effective in inhibiting the metastasis of cancer by preventing the migration of cancer cells and hindering the formation of new blood vessels (Korean registered Patent Publication No. 10-1323728, U.S registered Patent Publication No. 8716288, etc.).

However, a superior effect of mirror enantiomers of benproperine in preventing and treating cancer has not been yet identified, and it has been reported in Siwei Chen, et al., (J. Pharmacy and Pharmacology, 56(2): 277-280, 2004.02) that the anti-tussive effect of benproperine is not significant among the mirror enantiomers thereof.

Under above circumstances, the present inventors have made extensive efforts to develop a method for effectively preventing and treating cancer by inhibiting the migration of cancer cells, thus confirming that (S)-(-)-benproperine exhibits superior effects in inhibiting the migration and invasion of cancer cells compared to racemates thereof, thereby completing the present invention.

### [Disclosure]

### [Technical Problem]

One objective of the present invention is to provide a pharmaceutical composition for preventing and treating cancer, comprising (S)-(-)-benproperine or a pharmaceutically acceptable salt thereof as an active ingredient.

Another objective of the present invention is to provide a method for preventing and treating cancer, comprising administering an effective dose of the pharmaceutical composition to a subject in need.

Still another objective of the present invention is to provide a feed composition for preventing or alleviating cancer, comprising (S)-(-)-benproperine or a pharmaceutically acceptable salt thereof as an active ingredient.

### [Technical Solution]

In a first aspect to achieve the objectives described above, the present invention provides a pharmaceutical

composition for preventing and treating cancer, comprising (S)-(-)-benproperine or a pharmaceutically acceptable salt thereof as an active ingredient.

(S)-(-)-benproperine is a compound represented by Formula 1 below.

[Formula 1]

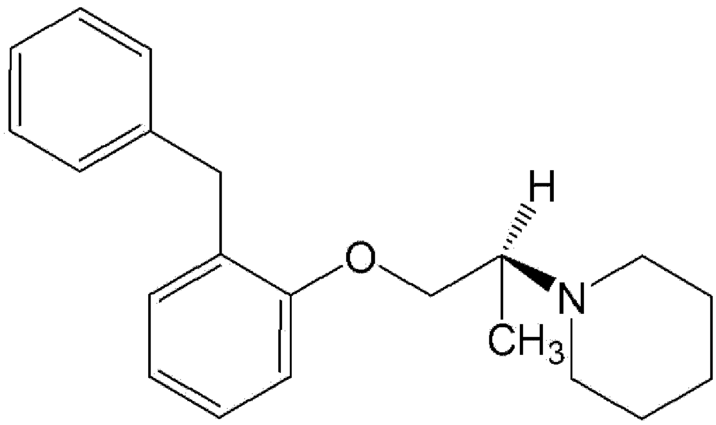

The (S)-(-)-benproperine can be easily prepared and used by a chemical synthesis according to a synthesis method known by one of ordinary skill in the art. In an example, the (S)-(-)-benproperine may be prepared by adding $K_2CO_3$ and N,N-dimethylformamide (DMF) to starting materials of 2-benzylphenol and (R)-(-)-propylene oxide. In another example, the (S)-(-)-benproperine may be prepared using (R)-1-(3-benzylphenoxy)propan-2-ol or (R)-(+)-1-(3-benzylphenoxy)propan-2-yl 4-methyl benzenesulfonate as starting materials. Also, commercially prepared (S)-(-)-benproperine may be purchased and used.

A pharmaceutically acceptable salt of (S)-(-)-benproperine may refer to a formulation that would not impair the biological activities and physical properties of (S)-(-)-benproperine.

The pharmaceutically acceptable salt may include, for example, a non-toxic acid addition salt produced by a free acid containing pharmaceutically acceptable anions. The free acid may include an acid addition salt produced by inorganic acids such as hydrochloric acid, sulfuric acid, nitric acid, phosphoric acid, hydrobromic acid, hydroiodic acid, and the like, organic carboxylic acids such as tartaric acid, formic acid, citric acid, acetic acid, trichloroacetic acid, trifluoroacetic acid, gluconic acid, benzoic acid, lactic acid, fumaric acid, maleic acid, salicylic acid, and the like, and sulfonic acids such as methanesulfonic acid, ethanesulfonic acid, benzenesulfonic acid, p- toluenesulfonic acid, and the like, but is not limited thereto. A pharmaceutically acceptable carboxylic acid salt may include a metal salt or an alkali earth metal salt produced by lithium, sodium, potassium, calcium, etc., an amino acid salt including lysine, arginine, guanine, etc., and an organic salt including dicyclohexylamine, N-methyl-D-glucamine, tris(hydroxymethyl)methylamine, diethanolamine, choline, triethylamine, etc.

The acid addition salt may have been prepared by a conventional method, for example, by dissolving (S)-(-)-benproperine in an excess amount of an acid aqueous solution, followed by a precipitation thereof using water-miscible organic solvents, for example, methanol, ethanol, acetone, or acetonitrile. Also, the acid addition salt may have been prepared by heating the same molar amount of (S)-(-)-benproperine and an acid or alcohol (for example, glycol monomethyl ether) in water and subsequently drying the mixture by evaporation, or a suction filtration of extracted salt.

Further, the pharmaceutically acceptable salt may include a pharmaceutically acceptable metal salt prepared using a base. The alkali metal salt or alkali earth metal salt may have been obtained, for example, by dissolving a compound in a solution containing excess alkali metal hydroxide or alkali earth metal hydroxide, and filtering insoluble salts, followed by evaporating and drying the filtrate.

The pharmaceutically acceptable salt may include any organic or inorganic acid addition salts in addition to the salts enumerated above.

In an example of the present invention, it was confirmed that the (S)-(-)-benproperine showed inhibitory activity against the migration of colon cancer cells, pancreatic cancer cells, and melanoma cells, which are representative cancer cell lines, and specifically, it was shown that the (S)-(-)-benproperine exhibited inhibitory activity against cancer metastasis which was active at a concentration that is more than twice as low as the concentration of a racemate thereof (FIGS.

3a, 3b). In another example of the present invention, it was also confirmed that the inhibitory effect of (S)-(-)-benproperine against the invasion of cancer cells was more significant at low concentration than that of the racemate (FIGS. 5a, 5b). Accordingly, the pharmaceutical composition of the present invention is effective in inhibiting the migration and invasion of cancer cells, and thus may be effectively used for the prevention and treatment of various cancers.

The cancers may refer to a state of destmying or modifying the original structures, in which the cancer cells have unregulated functions in normal cell division, cell differentiation, and cell apoptosis, and thus are abnormally hyper-proliferated, thereby invading surrounding tissues and organs, foiming lumps. The cancers are classified as benign, which merely reside in the site of occurrence, and malignant, which have spread to other parts of the body from the site of occurrence.

The examples of the cancers include colorectal cancer, pancreatic cancer, gastric cancer, liver cancer, breast cancer, cervical cancer, thyroid cancer, parathyroid cancer, lung cancer, non-small cell lung cancer, prostate cancer, gallbladder cancer, bile duct cancer, non-Hodgkin's lymphoma, Hodgkin's lymphoma, blood cancer, bladder cancer, renal cancer, ovarian cancer, melanoma, colon cancer, bone cancer, skin cancer, head cancer, uterine cancer, rectal cancer, brain tumor, anal cancer, fallopian tube carcinoma, endometrial carcinoma, vaginal cancer, vulva carcinoma, esophageal cancer, small intestine cancer, endocrine cancer, adrenal cancer, soft tissue sarcoma, urethral cancer, penis cancer, ureter cancer, renal cell carcinoma, renal pelvic carcinoma, central nervous system tumor, primary central nervous system lymphoma, spinal cord tumor, brainstem glioma, and pituitary adenoma, but are not limited thereto. The melanoma may occur in skin, eyes, mucous membranes, or the central nervous system.

Migration of the cancer cells, which is developed from the spread of cancer cells via blood circulation and lymphatic circulation, generally develops a new tumor after the cancer cells have spread to other organs via blood circulation.

Invasion of the cancer cells, which refers to penetration of cancer cells into a surrounding tissue from the site of occurrence, followed by proliferation, refers to direct migration of cancer cells into a neighboring tissue and infiltrating thereinto.

The term "prevention" used herein refers to any action that would inhibit or delay the pathogenesis of cancer by administering (S)-(-)-benproperine or a pharmaceutically acceptable salt thereof, according to the present invention, to a subject.

The term "treatment" used herein refers to any action that would improve or benefit the symptoms of cancer by administering the composition to a subject suspected of having the onset of cancer.

The unit dose of the pharmaceutical composition may be from 1 mg/kg to 100 mg/kg. The unit dose of pharmaceutical composition of the present invention may show effectiveness compared to the racemate having the same unit dose even at a low unit dose.

The pharmaceutical composition of the present invention may further include a pharmaceutically acceptable carrier, an excipient, or a diluting agent.

The term "pharmaceutically acceptable carrier" used herein refers to a carrier or diluting agent that would not hinder the biological activities and the properties of a compound administered, without irritating an organism. The types of carrier suitable for the present invention are not specifically limited, and any carrier can be used as long as it is conventionally used in the technical field of known art and pharmaceutically acceptable. The non-limiting examples of the carrier include saline, sterile water, Ringer's solution, buffered saline, an albumin injection solution, a dextrose solution, a maltodextrin solution, glycerol, ethanol, and the like. They may be employed either singly or in combination. Also, other conventional additives such as antioxidants, buffers, and/or bacteriostatic agents may be added to be used, if needed.

The pharmaceutical composition may be formulated to be prepared in the form of a unit volume or by delivering into a high-volume container. For example, the pharmaceutical composition may exist in one of the formulations selected from the group consisting of tablets, pills, powders, granules, capsules, suspensions, solutions, emulsions, syrups, sterilized aqueous solutions, non-aqueous solvents, suspensions, emulsions, lyophilization formulations, and suppositories, or may exist in various formulations of oral or parenteral types. When formulated, the formulations may be prepared using a diluting agent or an excipient, such as commonly-used fillers, weighting agents, bonding agents, wetting agents, disintegrating agents, and surfactants. The solid formulations for oral administration include tablets, pills, powders, granules, and capsules, and such solid formulations may be prepared by mixing at least one compound with at least one excipient, such as starch, calcium carbonate, sucrose or lactose, gelatin, and the like. Also, in addition to simple excipients, lubricants, such as magnesium stearate and talc, may also be used. Suspensions, solutions, emulsions, syrups, and the like are applied as the liquid formulations for oral administration, and may include various excipients, such as wetting agents, sweeteners, flavoring agents, and preservatives, in addition to the commonly used simple diluting agents water and liquid paraffin. The formulations for parenteral administration include sterile aqueous solutions, non-aqueous solvents, suspensions, emulsions, lyophilization formulations, and granules. Examples of the non-aqueous solvents and suspensions include vegetable oil, such as propylene glycol, polyethylene glycol, and olive oil, and an injectable ester, such as ethyl oleate. Witepsol, macrogol, tween 61, cacao butter, laurinum, and glycerol gelatin may

be used as the suppository base.

The pharmaceutical composition of the present invention may even be used as a single formulation. Alternatively, it may be used by preparing as a complex formulation further containing at least one type of anticancer drug.

The anticancer drug may be selected from a group consisting of DNA alkylating agents, anticancer antibiotics, and plant alkaloids, but is not limited thereto. For example, the anticancer drug may be at least one selected from the group consisting of mechloethamine, chlorambucil, phenylalanine, mustard, cyclophosphamide, ifosfamide, carmustine (BCNU), lomustine (CCNU), streptozotocin, busulfan, thiotepa, cisplatin, carboplatin, dactinomycin (actinomycin D), doxorubicin (adriamycin), daunorubicin, idarubicin, mitoxantrone, plicamycin, mitomycin, C bleomycin, vincristine, vinblastine, paclitaxel, docetaxel, etoposide, teniposide, topotecan, and iridotecan.

In another aspect, the present invention provides a method for preventing and treating cancer, including administering an effective dose of the pharmaceutical composition to a subject in need.

The pharmaceutical composition may refer to a pharmaceutical composition for preventing and treating cancer, including the foresaid (S)-(-)-benproperine or a pharmaceutically acceptable salt thereof as an active ingredient.

The term "subject" used herein may refer to any animals including humans who have or are at risk of developing cancer. The animals may include not only humans, but also mammals, such as cattle, horses, sheep, pigs, goats, camels, antelopes, dogs, cats, etc., which are in need of treatment for similar symptoms, but are not limited thereto.

Specifically, the method of the present invention for preventing and treating includes administering an effective dose of the composition to a subject who has or is at risk of developing cancer.

The "effective dose", which is a pharmaceutically effective dose, may refer to an amount that is sufficient to treat a disease at a reasonable benefit/risk ratio applicable to any medical treatment, and the level thereof may be determined depending on factors including the type of subject, the severity of disease, the patient's age and sex, drug activity, drug sensitivity, the duration of administration, the route of administration, the emission ratio, the duration of treatment, and drugs used in combination, and other factors known in the medical field. For example, the amount of composition administered may be from 0.001 to 1000 mg/kg, preferably, from 0.05 to 200 mg/kg, more preferably from 0.1 to 100 mg/kg, and may be administered once or multiple times a day. The composition of the present invention may be administered as an individual therapeutic agent or in combination with other therapeutic agents, and may be administered sequentially or coincidently with conventional therapeutic agents. In addition, the composition may be administered once or multiple times.

Considering all factors described above, it is important to administer the least amount of the composition for a maximum effect without causing any side effects, and the amount may be readily determined by one of ordinary skill in the art. The preferred dose of the composition of the present invention may vary depending on the patient's condition and body weight, the degree of disease, a drug form, and the route and duration of administration. The composition may be administered once or multiple times a day. Also, the amount administered may vary depending on various factors such as a specific composition used in combination or coincident with the composition of the present invention, and similar factors well known in the medical field. The composition may be administered to various mammals, such as rats, domestic animals, and humans via various routes, and the mode of administration may include any conventional methods known in the art, but preferably via a parenteral administration.

The term "administration" used herein refers to an introduction of the pharmaceutical composition of the present invention to a patient by an appropriate method, and the route of administration of the composition of the present invention may include various routes such as oral or parenteral administration as long as it can reach the desired tissue.

The mode of administration of the pharmaceutical composition of the present invention is not specifically limited, but may follow the conventional methods used in the technical field of known art. The non-limiting examples of the mode of administration include oral administration or parenteral administration. The pharmaceutical composition according to the present invention may be prepared as various formulations depending on the desired mode of administration.

The frequency of administration of the composition of the present invention is not specifically limited, but it may be administered once a day or multiple times in a divided dose.

In an example of the present invention, it was confirmed that (S)-(-)-benproperine showed inhibitory activity against the migration of colorectal cancer cells, pancreatic cancer cells, and melanoma cells, which are representative cancer cell lines, and specifically, it was shown that the (S)-(-)-benproperine exhibited inhibitory activity against cancer metastasis which was active at a concentration that is more than twice as low as the concentration of the racemate thereof (FIGS. 3a, 3b). In another example of the present invention, it was also confirmed that the inhibitory effect of (S)-(-)-benproperine against the invasion of cancer cells was more significant than that of the racemate (FIGS. 5a, 5b). Accordingly, the pharmaceutical composition of the present invention is effective in inhibiting the migration and invasion of cancer cells, and thus may be effectively used for the prevention and treatment of various cancers in animals including humans.

In still another aspect, the present invention provides a feed composition for preventing or alleviating cancer, including (S)-(-)-benproperine or a pharmaceutically acceptable salt thereof as an active ingredient.

The term "feed" used herein refers to any suitable natural or artificial diet, a single meal, and the like that an

animal consumes, ingests, and digests, or the ingredients of a single meal. The feed composition may include feed additives. The feed additives are considered as supplementary feed under feed control act.

The types of the feed additives are not specifically limited, but any feed that is conventionally used in the technical field of known art may be used. The non-limiting examples of the feed include vegetable feeds, such as cereals, nuts, food processed by-products, millet, fibers, pharmaceutical by-products, fats, starches, gourds, or grain by-products, etc., and animal feed, such as protein, inorganic substances, fats, minerals, single cell proteins, animal planktons, or food, etc. They may be used either singly or in combination.

The terms (S)-(-)-benproperine, cancer, prevention, or alleviation used herein are the same as described above.

In an example of the present invention, it was confirmed that (S)-(-)-benproperine showed inhibitory activity against the migration of colorectal cancer cells, pancreatic cancer cells, and melanoma cells, which are representative cancer cell lines, and specifically, it was shown that the (S)-(-)-benproperine exhibited inhibitory activity against cancer metastasis which was active at a concentration that is more than twice as low as the concentration of racemates thereof (FIGS. 3a, 3b). In another example of the present invention, it was also confirmed that the inhibitory effect of (S)-(-)-benproperine against the invasion of cancer cells was more significant than that of the racemates (FIGS. 5a, 5b). Accordingly, the feed composition of the present invention is effective in inhibiting the migration and invasion of cancer cells, and thus may be effectively used for the prevention and treatment of various cancers that may arise in animals.

**[Advantageous Effect]**

In the present invention, (S)-(-)-benproperine or a pharmaceutically acceptable salt thereof inhibits the migration and invasion of various cancer cells, and specifically exhibits a superior anticancer effect compared to racemates of benproperine, thus it can be effectively used for a pharmaceutical composition for preventing and treating cancer, and a method for preventing and treating using the same.

[Description of Drawings]

FIG. 1 shows $^{1}H$ NMR data of (S)-(-)-benproperine.

FIG. 2 shows graphs illustrating cell toxicity of DLD-1, a colorectal cancer cell line of (S)-(-)-benproperine, AsPc-1, a pancreatic cancer cell line thereof, and B16-BL6, a melanoma cell line thereof

FIG. 3a shows an inhibitory effect of colorectal cell DLD-1 against cell migration, when treated with 5 μM of benproperine racemate, (R)-(+)-benproperine (R-Ben), and (S)-(-)-benproperine (S-Ben), and FIG. 3b shows comparative results showing inhibitory effects of DLD-1 against cell migration when treated with benproperine racemate and (S)-(-)-benproperine (S-Ben) of varying concentrations (0.5 μM, 1 μM, and 2 μM).

FIG. 4 shows an inhibitory effect of MCF10A, normal cells, against cell migration when treated with 10 μM of benproperine racemate, (R)-(+)-benproperine (R-Ben), and (S)-(-)-benproperine (S-Ben).

FIG. 5a shows an inhibitory effect of DLD-1, a colorectal cell line, against cell invasion when treated with 5 μM of benproperine racemate, (R)-(+)-benproperine (R-Ben), and (S)-(-)-benproperine (S-Ben), and FIG. 5b shows an inhibitory effect of DLD-1 against cell invasion when treated with benproperine racemate and (S)-(-)-benproperine (S-Ben) of varying concentrations (0.5 μM, 1 μM, and 2 μM).

[Best Mode]

Hereinafter, the present invention will be described in more detail with reference to the following examples, comparative examples, and experimental examples. However, the following examples, comparative examples, and experimental examples are provided for illustrative purposes only, and the scope of the present invention should not be limited thereto in any manner.

**EXAMPLE 1: Synthesis of (S)-(-)-benproperine**

(S)-(-)-benproperine was synthesized in the same manner as described in Reaction 1 below.

[Reaction 1]

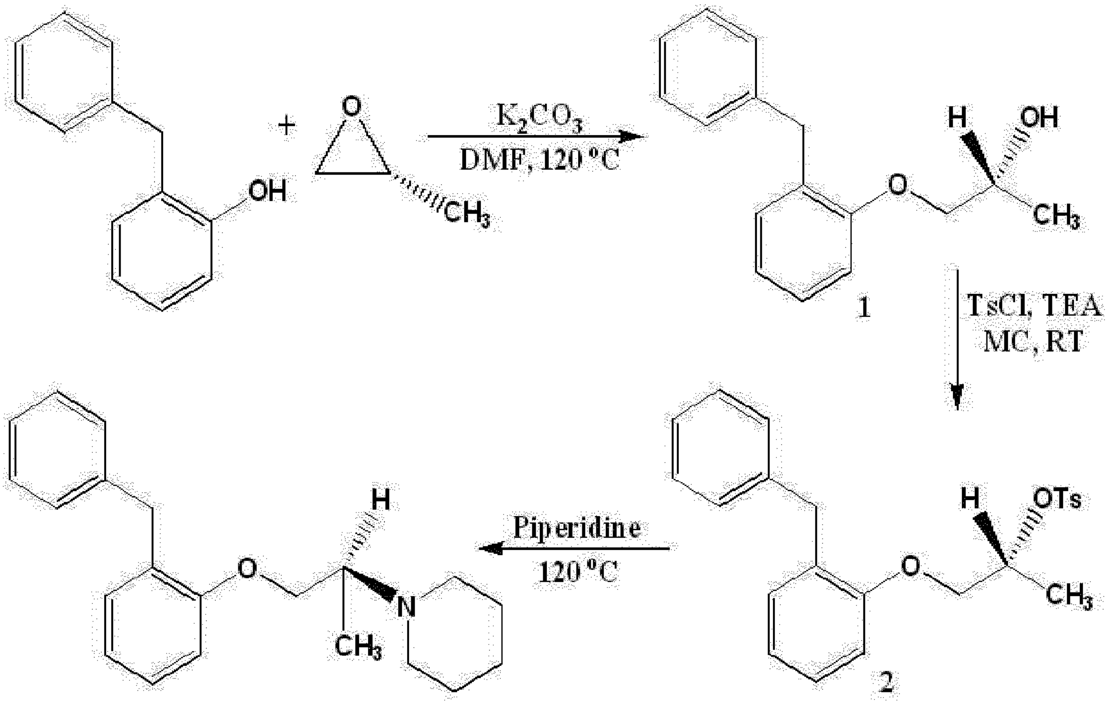


(S)-(-)-Benproperine

**(1) Synthesis of Compound 1 [(R)-1-(3-benzylphenoxy)propan-2-ol]**

1.2 g (8.68 mmol) of $K_2CO_3$ was added to 5 mL (0.85 M) of N,N-dimethylformamide (DMF) and stirred. After 5 minutes, 400 mg (2.17 mmol) of 2-benzylphenol was added thereto at room temperature. After 30 minutes, 0.9 mL (13.91 mmol) of (R)-(-)-propylene oxide was rapidly added thereto using a syringe. The resultant was then heated to 120°C and stirred for 17 hours. After cooling the resultant at room temperature, water was added to terminate the reaction, and the resultant was extracted with ethyl acetate and water three times. The organic layer was then washed with water three times, and further washed with saline water once. The resultant was dried over $MgSO_4$, filtered, and concentrated under reduced pressure. The resulting concentrate was purified by column chromatography (EA:Hex=1:9) to obtain compound 1 [(R)-1-(3-benzyl phenoxy)propan-2-ol, 970 mg, 92.4%, bright yellow oil]. The $^1$H-NNM analysis of the compound 1 is shown below.

$^1$HNMR (300MHz, CDCl3)δ7.30-7.15 (m, 7H), 6.94 (td, J = 7.2,1.2 Hz, 1H), 6.81 (d, J = 8.7 Hz, 1H), 4.06-4.03 (m, 1H), 4.00 (d, J = 5.4 Hz, 2H), 3.89 (dd, J = 9.3,3.0 Hz, 1H), 3.66 (dd, J = 9.3, 7.8 Hz, 1H), 1.78 (d, J = 3.6 Hz, 1H), 1.18 (d, J = 6.0 Hz, 3H,);

$^{13}$CNMR (75MHz, CDCl3)δc 156.34, 141.22, 131.00, 129.18, 128.45, 128.26, 127.77, 126.01, 120.81,111.20,78.08,66.10,37.10,18.36.

**(2) Synthesis of compound 2 [(R)-(+}-1-(3-benzylphenoxy)propan-2-yl 4-methyl benzenesulfonate]**

The compound 1 (400 mg, 1.73 mmol) was dissolved in 7 mL (0.25 M) of methylene chloride (MC). Then, 0.59 mL (4.21 mmol) of triethylamine (TEA), 106 mg (0.87 mmol) of p-dimethylanimopyridine (DMAP), and 410 mg (2.25 mmol) of p-toluenesulfonyl chloride (TsCl) were sequentially added thereto and stirred for 16 hours. Water was then added to terminate the reaction, and the resultant was extracted with methylene chloride (MC) and water three times. Then the MC layer was washed with water twice, and further washed with saline water once. The resultant was dried over $MgSO_4$, filtered, and concentrated under reduced pressure. The resulting concentrate was purified by column chromatography (EA:Hex=1:9) to obtain compound 2 [(R)-1-(3-benzylphenoxy)propan-2-yl 4-methyl benzenesulfonate, 505 mg, 73.5%, brown oil]. The $^1$H NMR analysis of the compound 2 is shown below.

[a]$^{25}$D+78.7° (c 1, EtOH); $^1$H NMR (300 MHz) δ 7.78 (d, 2H, J = 8.1Hz), 7.27-7.11 (m, 8H), 7.03 (dd, J = 7.2,1.2 Hz, 1H), 6.88 (t, J = 7.2 Hz, 1H), 6.70 (d, 1H, J = 8.7Hz), 4.87 (m, 1H), 4.01 (dd, 1H, J = 10.5, 5.4 Hz), 3.89 (dd, 1H, J = 10.5, 5.4 Hz), 3.80 (s, 2H), 2.37 (s, 3H), 1.34 (d, 3H, J = 6.9Hz);

$^{13}$CNMR (75MHz) δc 155.6, 144.7, 140.7, 134.0, 130.5, 129.9, 129.7, 128.9, 128.2, 127.7, 127.3, 125.8,

121.1,111.1, 76.9, 69.7, 35.7, 21.5, 17.8.

**(3) Synthesis of (S)-(-)-benproperine**

The compound 2 (435 mg, 1.09 mmol) was dissolved in piperidine (0.65 mL, 6.59 mmol) and was refluxed at 120°C for 5 hours. Thereafter, the resultant was stirred for 12 hours at room temperature. The stirred reaction mixture was diluted with methanol and concentrated under pressure to remove excess piperidine. 43 mL of 1 M sodium hydroxide (NaOH) was then added to the reaction mixture to provide basic conditions, and the resultant was extracted with ether and water three times. The ether layer was then washed with saline water, and the resultant was dried over $MgSO_4$, filtered, and concentrated under reduced pressure. The resulting concentrate was purified by column chromatography (EA:Hex=1:1) to obtain (S)-(-)-benproperine (222 mg, 65.9%, brown oil). The $^1$H NMR analysis of (S)-(-)-benproperine is shown below.

$[a]^{25}$D-121.8°(c1,EtOH); $^1$H NMR (300 MHz) $\delta$ 7.29-7.07 (m, 7H), 6.87 (t, J = 7.2Hz, 2H), 4.07 (dd, 1H, J = 9.3, 4.8 Hz), 4.00 (s, 2H), 3.85 (dd, 1H, J = 9.0, 6.6 Hz), 2.97 (m, 1H), 2.55 (m, 4H), 1.56 (m, 4H), 1.42 (m, 2H), 1.11 (d, 3H, J = 6.6Hz);

$^{13}$CNMR (75MHz) $\delta$c 156.6, 141.0, 130.4, 129.5, 128.8, 128.1, 127.4, 125.7, 120.3, 111.1, 69.9, 58.9, 50.4, 36.1, 26.5, 24.7, 13.2.

The $^1$H NMR data of the (S)-(-)-benproperine, synthesized as described above, is shown in FIG. 1.

**Comparative Example 1: Synthesis of (R)-(+)- benproperine**

The synthesis of (R)-(+)-benproperine, another mirror enantiomer of (S)-(-)-benproperine, was carried out using a synthesis method different from that of (S)-(-)-benproperine and different starting materials in the same manner as described in Reaction 2 below.

[Reaction 2]

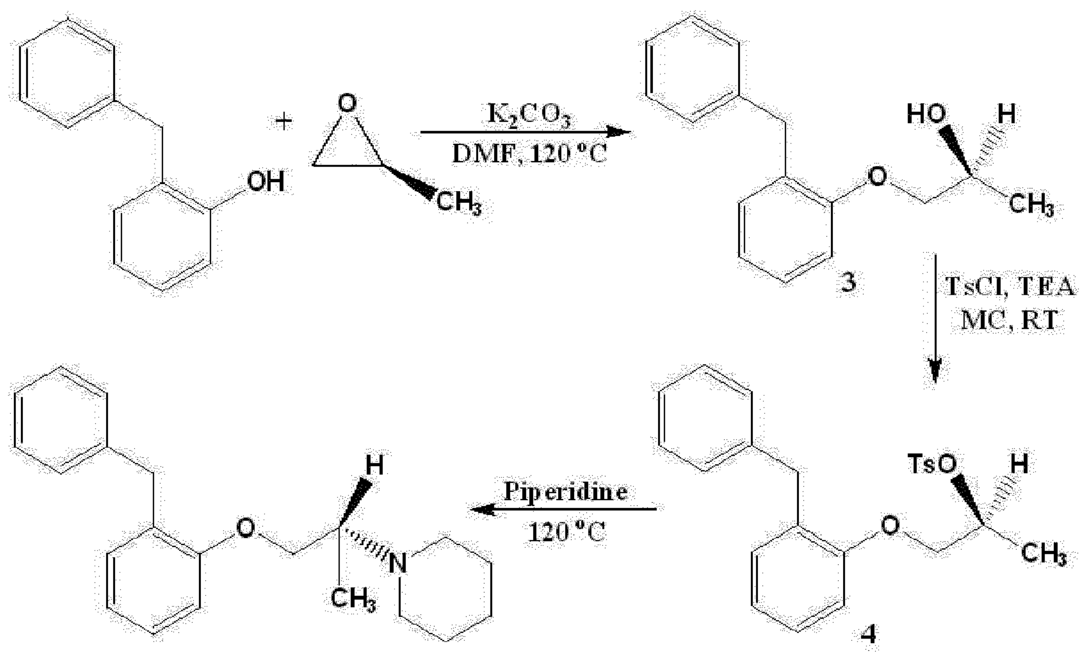


(R)-(+)-Benproperine

**(1) Synthesis of Compound 3 [(S)-1-(3-benzylphenoxy)propan-2-ol]**

1.2 g (8.68 mmol) of $K_2CO_3$ was added to 5 mL (0.85 M) of N,N-dimethylformamide (DMF) and stirred. After 5 minutes, 400 mg (2.17 mmol) of 2-benzylphenol was added thereto at room temperature. After 30 minutes, 0.9 mL

(13.91 mmol) of (S)-(-)-propylene oxide was rapidly added thereto using a syringe. The resultant was then heated to 120°C and stirred for 17 hours. After cooling the resultant to room temperature, water was added to terminate the reaction, and the resultant was extracted with ethyl acetate and water three times. The organic layer was then washed with water three times, and further washed with saline water once. The resultant was dried over $MgSO_4$, filtered, and concentrated under reduced pressure. The resulting concentrate was purified by column chromatography (EA:Hex=1:14) to obtain compound 3 [(S)-1-(3-benzylphenoxy)propan-2-ol, 340 mg, 64.8%, bright yellow oil]. The $^1$H-NNM analysis of the compound 3 is shown below.

$^1$HNMR (300MHz) δ 7.31-7.16 (m, 7H), 6.94 (td, J = 7.2, 1.2 Hz, 1H), 6.82 (d, J = 8.1 Hz, 1H), 4.06-4.04 (m, 1H), 4.00 (d, J = 5.4 Hz, 2H) 3.89 (dd, J = 9.9, 3.0 Hz, 1H), 3.67 (dd, J = 9.0, 7.2 Hz, 1H), 1.78 (d, J = 2.4 Hz, 1H), 1.18 (d, J = 6.6 Hz, 3H));

$^{13}$CNMR (75MHz) δc 156.3, 141.2, 131.0, 129.1, 128.4, 128.2, 127.7, 126.0, 120.8, 111.2, 78.0, 66.1, 37.1,18.3.

**(2) Synthesis of Compound 4 [(S)-(-)-1-(3-benzylphenoxy)propan-2-yl 4-methyl benzenesulfonate]**

The compound **3** (340 mg, 1.40 mmol) was dissolved in 7 mL (0.25 M) of methylene chloride (MC). Then, 0.59 mL (4.21 mmol) of triethylamine (TEA), 106 mg (0.87 mmol) of p-dimethylanimopyridine (DMAP), and 410 mg (2.25 mmol) of p-toluenesulfonyl chloride (TsCl) were sequentially added thereto and stirred for 16 hours. Water was then added to terminate the reaction, and the resultant was extracted with methylene chloride (MC) and water three times. Then the MC layer was washed with water twice, and further washed with saline water once. The resultant was dried over $MgSO_4$, filtered, and concentrated under reduced pressure. The resulting concentrate was purified by column chromatography (EA:Hex=1:12) to obtain compound **4** [(S)-1-(3-benzylphenoxy)propan-2-yl 4-methyl benzenesulfonate, 390 mg, 70.2%, brown oil]. The $^1$H-NNM analysis of the compound 4 is shown below.

[a]$^{25}$D-9.00°(c1,EtOH); $^1$H NMR (300MHz) δ 7.79-7.76 (m, 2H), 7.27-7.11 (m, 8H), 7.03 (dd, J = 7.8, 1.8 Hz, 1H), 6.88 (m, 1H), 6.70 (d, 1H, J = 8.1Hz), 4.87 (m, 1H), 3.96 (dd, 1H, J = 9.9,5.4 Hz), 3.89 (dd, 1H, J =10.5, 4.8 Hz), 3.81 (s, 2H), 2.36 (s, 3H), 1.34 (d, 3H, J = 6.0Hz);

$^{13}$C-NMR (75MHz) δc 155.6, 144.7, 140.7, 134.0, 130.5, 129.9, 129.7, 128.9, 128.2, 127.7, 127.3, 125.8,121.1, 111.1, 76.9, 69.8, 35.7, 21.5, 17.8.

**(3) Synthesis of (R)-(+)-benproperine**

The compound **4** (390 mg, 0.96 mmol) was dissolved in piperidine (0.57 mL, 5.76 mmol) and was refluxed at 120°C for 5 hours. Thereafter, the resultant was stirred for 12 hours at room temperature. The stirred reaction mixture was diluted with methanol and concentrated under pressure to remove excess piperidine. 43 mL of 1 M sodium hydroxide (NaOH) was then added to the reaction mixture to provide basic conditions, and the resultant was extracted with ether and water three times. The ether layer was then washed with saline water, and the resultant was dried over $MgSO_4$, filtered, and concentrated under reduced pressure. The resulting concentrate was purified by column chromatography (EA:Hex=1:1) to obtain (R)-(+)-benproperine (210 mg, 72.4%, brown oil). The $^1$H NMR analysis of the (R)-(+)-benproperine is shown below.

[a]$^{25}$D+ 15.94°(c1,EtOH); $^1$HNMR (400MHz) δ 7.27-7.14 (m, 7H), 7.08 (dd, J = 7.2, 1.2 Hz, 1H), 6.87 (m, 2H), 4.07 (dd, 1H, J = 8.8,4.4 Hz), 3.99 (s, 2H), 3.85 (dd, 1H, J = 9.2, 6.4 Hz), 2.97 (m, 1H), 2.55 (m, 4H), 1.55 (m, 4H), 1.41 (m, 2H), 1.11 (d, 3H, J = 6.8Hz);

$^{13}$CNMR (100MHz) δc 156.6, 141.0, 130.48, 129.6, 128.8, 128.1, 127.4, 125.7, 120.3, 111.1, 69.9, 58.9, 50.4, 36.1, 26.5, 24.7, 13.2.

**Experimental Example 1: A cytotoxicity assay of (S)-(-)-benproperine and (R)-(+)-benproperine**

To verify the cytotoxicity of (S)-(-)-benproperine, prepared in Example 1, DLD-1 cells (ATCC-CCL-221), a human colorectal cancer cell line, AsPc-1 cells (ATCC-CRL-1682), a pancreatic cancer cell line, B16-BL6 cells (ATCC-CRL-6475), a melanoma cell line, and normal cells of MCF10A (ATCC-CRL-10317) were cultured in an RPMI medium containing 10% fetal bovine serum (FBS) while maintaining a temperature at 37°C and 5% $CO_2$, and the cells were then detached from the medium using 0.05% trypsin-EDTA. Each well of a 96-well plate including the medium containing 10% FBS was inoculated with $4\times10^3$ cells, calculated with a hemocytometer, and the cells were cultured in an incubator containing 5% $CO_2$ at 37°C.

After 24 hours, the medium of each well was replaced with a new medium containing a control group (0.1% DMSO) and 5 μM of benproperine, prepared in either of Example 1 or Comparative Example 1. The cells in the new media were then cultured in an incubator containing 5% $CO_2$ at 37°C for 48 hours. Each well was then charged with 10 μL of WST-1 (Roche), culturing was continued for 2 hours, and absorbance was measured at 450 nm by ELISA Reader (Bio-Rad).

As a result, the (S)-(-)-benproperine did not exhibit the cytotoxicity against the normal cells of MCF10A as well as the cancer cells.

**Experimental Example 2: An inhibitory activity assay against the migration and invasion of cancer cells**

To analyze inhibitory activity against the migration and invasion of (S)-(-)-benproperine cancer cells, a cell migration and invasion assay was carried out using DLD-1 cells (ATCC-CCL-221), a PRL-3 over-expressed colorectal cancer cell line, using a transwell as described below.

First, DLD-1 cells (ATCC-CCL-221) were allowed to grow to about 90% in a culturing plate containing an RPMI medium, then removed from the medium and washed with PBS. The washed cells were treated with trypsin-EDTA and cultured in a $CO_2$ incubator at 37°C for 15 hours to detach the cells. Then an RPMI medium containing 10% FBS was added thereto, and the cells were collected in a Falcon tube and centrifuged to remove the medium. The thus-obtained cells were charged with an FBS-free RPMI medium and centrifuged to remove the medium, and these step were repeated twice. The thus-obtained cells were charged with an FBS-free RPMI medium and then suspended to measure the number of cells using a hemocytometer.

Thereafter, the transwell was placed on top of a 24-well plate and charged with cells of $8\times10^4$ cell/200 μL. The transwell was charged with 500 μL of an FBS-free RPMI medium containing the (S)-(-)-benproperine, prepared in Example 1, through the gap, and cultured in a $CO_2$ incubator at 37°C for 12 hours to 16 hours. After culturing, the transwell was turned upside down on a hand towel to remove the medium, then each well of the 24-well plate was charged with 500 μL of crystal violet (5 mg/mL in 20% MeOH). The transwell was placed back on top of the 24-well plate and incubated in a $CO_2$ incubator for 30 minutes. The stained transwell was washed with PBS and turned upside down on a hand towel to let the remaining solution infiltrate into the hand towel. A region where the cells had not moved was labeled as the top of a film by placing a cover slip on top of the film, and the cells that had not moved were wiped off with a cotton swab. A small amount of nail polish was applied to the edge of the cover slip, and a slide glass was placed on top to fix the cover slip. The thus-prepared sample was photographed using an inverted microscope (TE 300, Nikon, Japan) with a digital camera installed, and the number of migrated cells was calculated.

Subsequently, cell migration inhibition was calculated via Mathematical Formula 1 shown below.

[Mathematical Formula 1]

$$\text{Cell Migration Inhibition (\%)} = 100-\left[\frac{\text{Number of migrated cells in sample-treated group}}{\text{Number of migrated cells in control group}}\times100\right]$$

According to the Mathematical Formula 1 above, "the number of migrated cells in sample-treated group" refers to a number of migrated cells measured after treating DLD-1 with (S)-(-)-benproperine (S-Benp) prepared in Example 1, (R)-(+)- benproperine (R-Benp) prepared in Comparative Example 1, or a mixed racemate having a 1:1 weight ratio of (S)-(-)-benproperine to (R)-(+)- benproperine, and "the number of migrated cells in control group" refers to a number of migrated cells measured after treating DLD-1 with 1% dimethyl sulfoxide (DMSO) alone, instead of benproperine.

As a result, as shown in FIG. 3a, the racemate (Benp) inhibited the cell migration by 71% compared to the DMSO-treated control group, whereas the (S)-(-)-benproperine (S-Benp) inhibited the cell migration by 89% in DLD-1 cells, a colorectal cancer cell line. Also, as shown in FIG. 3b, the racemate (Benp) inhibited the cell migration by only 3% at a concentration of 0.5 μM compared to the DMSO-treated control group, whereas the (S)-(-)-benproperine inhibited the cell migration by 32%. Specifically, the (S)-(-)-benproperine inhibited the cell migration by 52% at a concentration of 1 μM, whereas the racemate (Benp) showed inhibitory activity against the cell migration by 53% at a concentration of 2 μM, which is twice the concentration of (S)-(-)-benproperine. Accordingly, the (S)-(-)-benproperine exhibited twice as much inhibitory activity against the cell migration as the racemate. Such results imply that the (S)-(-)-benproperine may exhibit inhibitory activity against the active cancer metastasis even at a concentration that is twice as low as the concentration of the racemate.

Meanwhile, as shown in FIG. 4, the (S)-(-)-benproperine did not inhibit the migration of MCF10A cells, which are normal cells.

Also, as shown in FIG. 5a, the racemate (Benp) inhibited the cell invasion by 73% compared to the DMSO-treated control group, whereas the (S)-(-)-benproperine (S-Benp) inhibited the cell invasion by 90% in DLD-1 cells, a colorectal cancer cell line.

Further, as shown in FIG. 5b, the racemate (Benp) inhibited the cell invasion by 21% at a concentration of 1 μM, compared to the DMSO-treated control group, whereas the (S)-(-)-benproperine (S-Benp) inhibited the cell invasion by 36%. Specifically, it was confirmed that the (S)-(-)-benproperine (S-Benp) inhibited the cell invasion by 57% at a low concentration of 2 μM, whereas the racemate exhibited a significantly low cell invasion inhibition rate (36%). Accordingly, the results imply that the (S)-(-)-benproperine shows a strong inhibition against cell invasion compared to the racemate.
As described above, the (S)-(-)-benproperine is effective in inhibiting not only the migration of cancer cells but also the invasion of cancer cells. Accordingly, it is implied that the composition including the (S)-(-)-benproperine or a pharmaceutically acceptable salt thereof may be effectively used for prevention and treatment of various cancers.

**Experimental Example 3: Acute toxicity experiment of oral administration on rats**

6-week-old specific pathogen free SD type rats were divided into groups, where each group contains two rats. The (S)-(-)-benproperine, prepared in Example 1, was dissolved in water for injection, and orally administered to each rat once at a volume of 1000 mg/kg. After administering a test material, the possibilities of animal death, clinical symptoms, and weight fluctuation were observed to carry out hematological and blood biochemical examinations, and an autopsy was conducted to visually detect the presence of any abnormality in abdominal organs and thoracic organs.
As a result, all animals in which the testing material was administered showed noteworthy clinical symptoms, but none died nor showed any toxicity in weight fluctuation, hematological and blood biochemical examinations, post-mortem reports, etc.
Accordingly, since the (S)-(-)-benproperine does not exhibit toxicity until the volume reaches to 1000 mg/kg in all rats, it was determined to be a safe material having a minimum lethal oral dose (LD50) of 1000 mg/kg.

**Formulation Example 1: Preparation of powder**

| | |
|---|---|
| (S)-(-)-benproperine* | 20 mg |
| Lactose | 100 mg |
| Talc | 10 mg |

(* refers to (S)-(-)-benproperine, a pharmaceutically acceptable salt thereof or a derivate thereof; the same as below)
The powder was prepared by mixing the ingredients listed above and filling into an airtight package according to a conventional method for preparing powder.

**Formulation Example 2: Preparation of tablets**

| | |
|---|---|
| (S)-(-)-benproperine* | 10 mg |
| Cornstarch | 100 mg |
| Lactose | 100 mg |
| Magnesium stearate | 2 mg |

The tablets were prepared by mixing the ingredients listed above, followed by tabletting according to a conventional method for preparing tablets.

**Formulation Example 3: Preparation of capsules**

| | |
|---|---|
| (S)-(-)-benproperine* | 10.0 mg |
| Crystalline cellulose | 3.0 mg |
| Lactose | 14.8 mg |
| Magnesium stearate | 0.2 mg |

The capsules were prepared by mixing the ingredients listed above, followed by filling into gelatin capsules according to a conventional method for preparing capsules.

**Formulation Example 4: Preparation of injections**

| | |
|---|---|
| (S)-(-)-benproperine* | 10 mg |
| Mannitol | 180 mg |
| Injectable sterile distilled water | 2974 mg |
| $Na_2HPO_4 \cdot 12H_2O$ | 26 mg |

The injections were prepared by mixing the ingredients listed above with injectable sterile distilled water so that each injection contained 2 mL of the ingredient per ampule according to a conventional method for preparing injections.

**Formulation Example 5: Preparation of liquid formulations**

| | |
|---|---|
| (S)-(-)-benproperine* | 10 mg |
| Isomerized glucose syrup | 10 g |
| Mannitol | 5 g |
| Distilled water | to 100 mL |

The liquid formulations were prepared by mixing the ingredients listed above, adding distilled water to adjust the total volume to 100 mL, and filling into brown bottles according to a conventional method for preparing liquid formulations.

Although the preferred embodiments of the present invention have been disclosed for illustrative purposes, those skilled in the art will appreciate that various modifications, additions and substitutions are possible, without departing from the scope and spirit of the invention as disclosed in the accompanying claims.

## Claims

1. A pharmaceutical composition comprising (S)-(-)-benproperine represented by Formula 1 below or a pharmaceutically acceptable salt thereof as an active ingredient, for use in the prevention or treatment of cancer.

[Formula 1]

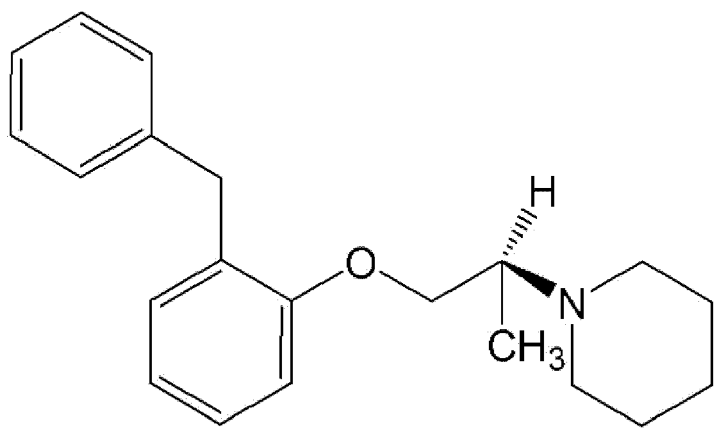

2. The pharmaceutical composition of claim 1, wherein the composition further comprises a pharmaceutically acceptable carrier, an excipient, or a diluting agent.

3. The pharmaceutical composition of claim 1, wherein the composition suppresses migration and invasion of a cancer cell.

4. The pharmaceutical composition of claim 1, wherein the cancer is colorectal cancer, pancreatic cancer, gastric cancer, liver cancer, breast cancer, cervical cancer, thyroid cancer, parathyroid cancer, lung cancer, non-small cell lung cancer, prostate cancer, gallbladder cancer, bile duct cancer, non-Hodgkin's lymphoma, Hodgkin's lymphoma, blood cancer, bladder cancer, renal cancer, ovarian cancer, melanoma, colon cancer, bone cancer, skin cancer, head cancer, uterine cancer, rectal cancer, brain tumor, anal cancer, fallopian tube carcinoma, endometrial carcinoma, vaginal cancer, vulva carcinoma, esophageal cancer, small intestine cancer, endocrine cancer, adrenal cancer, soft tissue sarcoma, urethral cancer, penis cancer, ureter cancer, renal cell carcinoma, renal pelvic carcinoma, central nervous system tumor, primary central nervous system lymphoma, spinal cord tumor, brainstem glioma, and pituitary adenoma.

5. The pharmaceutical composition of claim 4, wherein the melanoma occurs in skin, eyes, mucous membranes, or the central nervous system.

6. The pharmaceutical composition of claim 1, wherein the composition further comprises at least one type of anticancer drug.

7. The pharmaceutical composition of claim 6, wherein the anticancer drug is at least one selected from the group consisting of DNA alkylating agents, anticancer antibiotics, and plant alkaloids.

8. The pharmaceutical composition of claim 6, wherein the anti-cancer drug is at least one selected from the group consisting of mechloethamine, chlorambucil, phenylalanine, mustard, cyclophosphamide, ifosfamide, carmustine (BCNU), lomustine (CCNU), streptozotocin, busulfan, thiotepa, cisplatin, carboplatin, dactinomycin (actinomycin D), doxorubicin (adriamycin), daunorubicin, idarubicin, mitoxantrone, plicamycin, mitomycin, C bleomycin, vincristine, vinblastine, paclitaxel, docetaxel, etoposide, teniposide, topotecan, and iridotecan.

【FIG. 1】

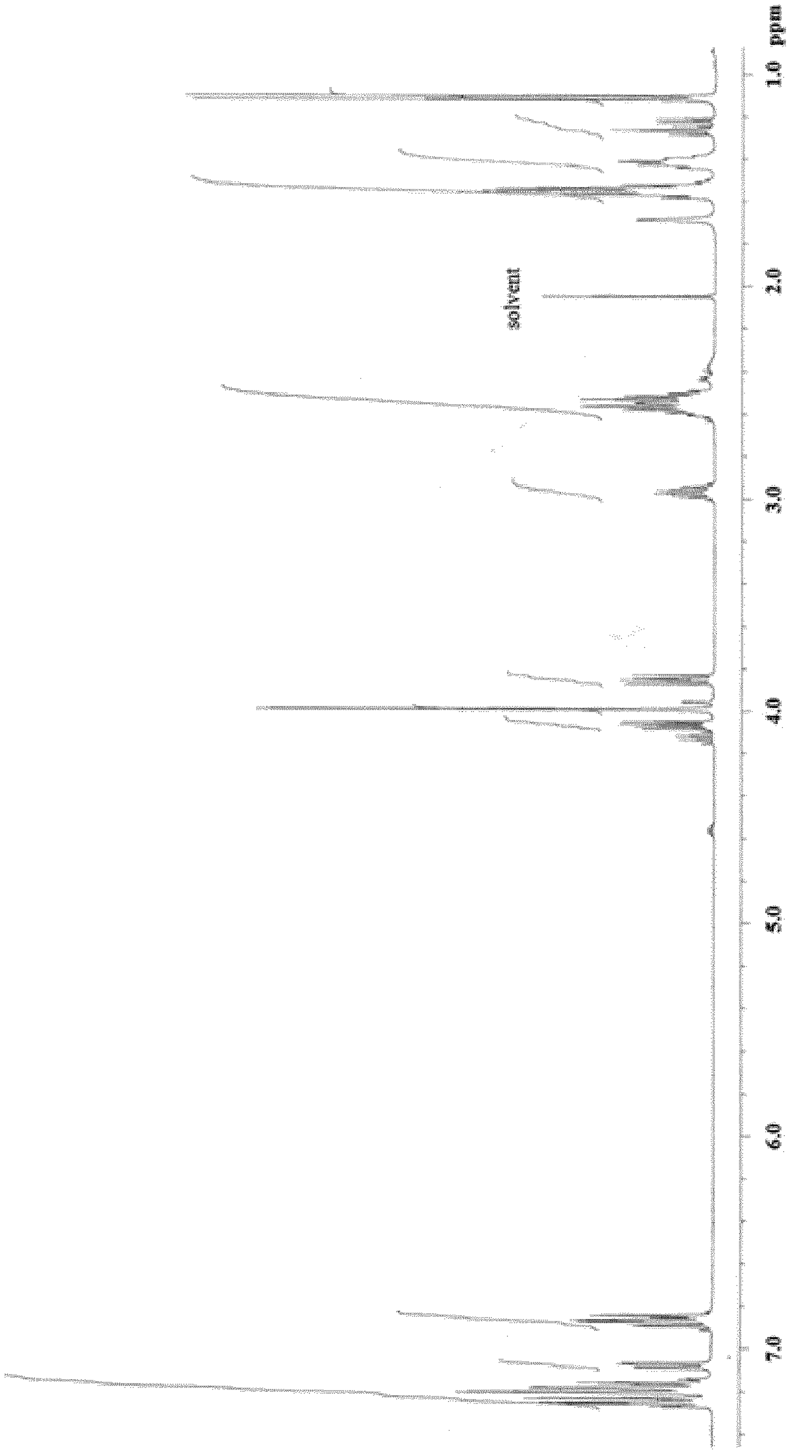

【FIG. 2】

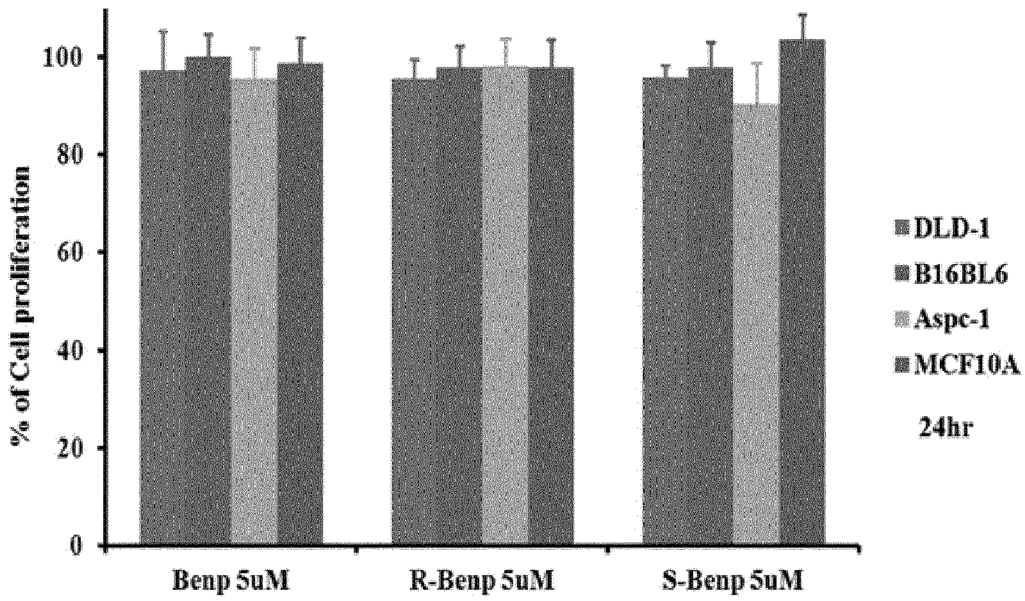
100
80
60
40
20
0
% of Cell proliferation
Benp 5uM
R-Benp 5uM
S-Benp 5uM
DLD-1
B16BL6
Aspc-1
MCF10A
24hr

【FIG. 3a】

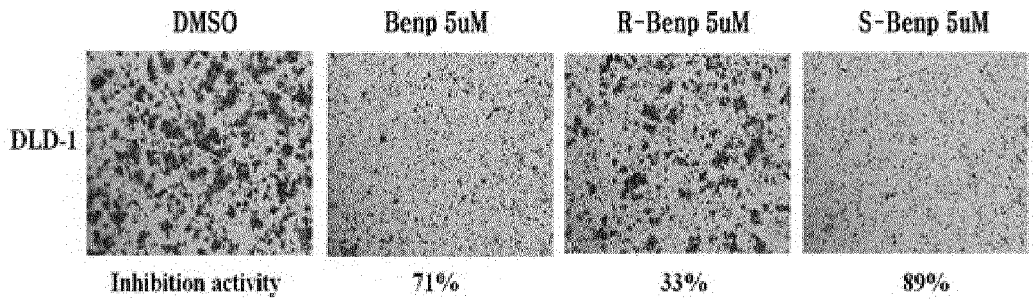
DMSO
Benp 5uM
R-Benp 5uM
S-Benp 5uM
DLD-1
Inhibition activity
71%
33%
89%

【FIG. 3b】

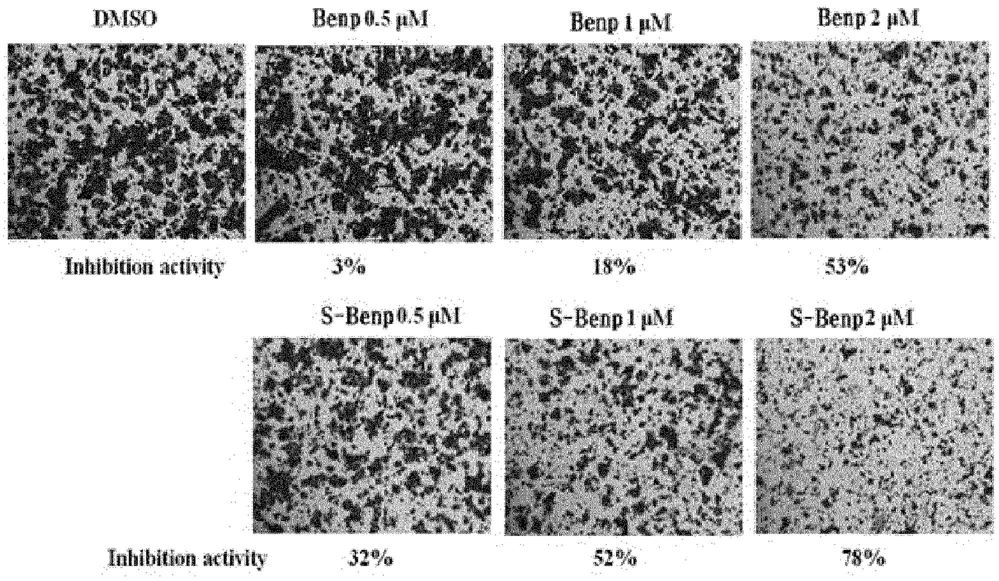


【FIG. 4】

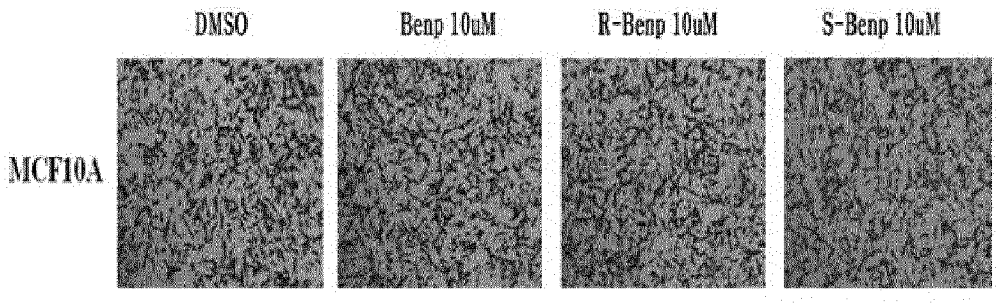


【FIG. 5a】

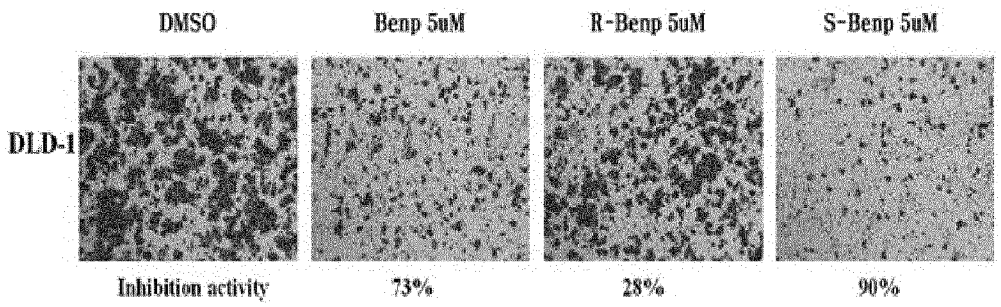

【FIG. 5b】

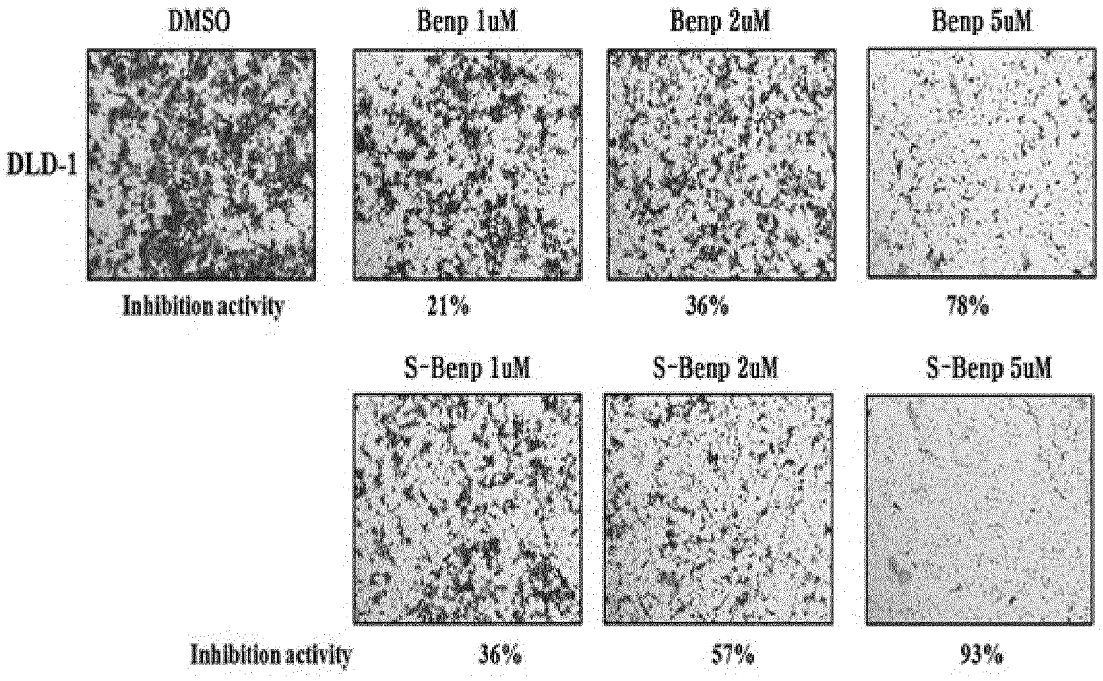
DMSO
Benp 1uM
Benp 2uM
Benp 5uM
DLD-1
Inhibition activity
21%
36%
78%
S-Benp 1uM
S-Benp 2uM
S-Benp 5uM
Inhibition activity
36%
57%
93%

Europäisches Patentamt
European Patent Office
Office européen des brevets

## EUROPEAN SEARCH REPORT

Application Number
EP 15 18 8301

| Category | Citation of document with indication, where appropriate, of relevant passages | Relevant to claim | CLASSIFICATION OF THE APPLICATION (IPC) |
|---|---|---|---|
| Y | US 2013/245068 A1 (KWON BYOUNG MOG [KR] ET AL) 19 September 2013 (2013-09-19)<br>* claims 1-3 *<br>* paragraph [0094] *<br>* page 9; examples 5,6 * | 1-8 | INV.<br>A61K31/4453<br>A61P35/00 |
| Y | SIWEI CHEN ET AL: "Anti-tussive activity of benproperine enantiomers on citric-acid-induced cough in conscious guinea-pigs",<br>JOURNAL OF PHARMACY AND PHARMACOLOGY,<br>vol. 56, no. 2,<br>1 February 2004 (2004-02-01), pages 277-280, XP55233842,<br>LONDON; GB<br>ISSN: 0022-3573, DOI: 10.1211/0022357022719<br>* page 277, paragraph 1 *<br>* page 280, column 1 * | 1-8 | |
| Y | LISE SCHJELDERUP ET AL: "The absolute configuration of benproperinium dihydrogen phosphate, an antitussive drug",<br>CHIRALITY.,<br>vol. 1, no. 1, 1 January 1989 (1989-01-01), pages 86-88, XP55233961,<br>US<br>ISSN: 0899-0042, DOI: 10.1002/chir.530010115<br>* page 86, column 1, paragraph 1 * | 1-8 | TECHNICAL FIELDS SEARCHED (IPC)<br>A61K |
| Y | DU ZONG-MIN ET AL: "Enantioselective pharmacokinetics of benproperine in healthy volunteers",<br>YAOXUE XUEBAO,<br>vol. 35, no. 12, December 2000 (2000-12), pages 909-912, XP8178366,<br>ISSN: 0513-4870<br>* abstract * | 1-8 | |

The present search report has been drawn up for all claims

2

| Place of search | Date of completion of the search | Examiner |
|---|---|---|
| The Hague | 8 December 2015 | Bonzano, Camilla |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

# ANNEX TO THE EUROPEAN SEARCH REPORT ON EUROPEAN PATENT APPLICATION NO. EP 15 18 8301

This annex lists the patent family members relating to the patent documents cited in the above-mentioned European search report.
The members are as contained in the European Patent Office EDP file on
The European Patent Office is in no way liable for these particulars which are merely given for the purpose of information.

08-12-2015

| Patent document cited in search report | Publication date | Patent family member(s) | Publication date |
|---|---|---|---|
| US 2013245068 A1 | 19-09-2013 | EP 2638910 A2 | 18-09-2013 |
| | | JP 2013544815 A | 19-12-2013 |
| | | KR 20120050918 A | 21-05-2012 |
| | | US 2013245068 A1 | 19-09-2013 |
| | | WO 2012064150 A2 | 18-05-2012 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- KR 101323728
- US 8716288 B


### Non-patent literature cited in the description


- **MARINA BACAC ; IVAN STAMENKOVIC.** *Annual Review of Pathology: Mechanism of Disease,* 2008, vol. 3, 221-247
- **PETER FRIEDL et al.** *Nature Cancer Review,* 2003, vol. 3, 362
- **SIWEI CHEN et al.** *Journal of Pharmacy and Pharmacology,* 2004, vol. 56, 277-280
- **SIWEI CHEN et al.** *J. Pharmacy and Pharmacology,* February 2004, vol. 56 (2), 277-280